# EUROPEAN PATENT APPLICATION

(11) **EP 4 397 670 A1**
(43) Date of publication of application: **10.07.2024**
(21) Application number: 22863467.1
(22) Date of filing: 30.08.2022
(51) Int. Cl.: C07J 9/00

(54) **STEROIDAL COMPOUND, PREPARATION METHOD THEREFOR AND APPLICATION THEREOF**

(30) Priority: 31.08.2021 CN 202111013742
(71) Applicant: Guangzhou Ocusun Ophthalmic Biotechnology Co., Ltd., Guangzhou, Guangdong 511400 (CN); Ocusun Ophthalmic Pharmaceutical (Guangzhou) Co., Ltd., Guangzhou, Guangdong 511400 (CN)
(72) Inventor: YU, Chuiliang, Guanzhou, Guangdong 511400 (CN); LI, Xiaolin, Guanzhou, Guangdong 511400 (CN); WANG, Yandong, Guanzhou, Guangdong 511400 (CN); LAN, Xiaobing, Guanzhou, Guangdong 511400 (CN); HAO, Fei, Guanzhou, Guangdong 511400 (CN); SU, Yingxue, Guanzhou, Guangdong 511400 (CN); HE, Haiying, Guanzhou, Guangdong 511400 (CN); WU, Meirong, Guanzhou, Guangdong 511400 (CN)
(74) Representative: Murgitroyd & Company
(86) International application number: PCT/CN2022/115896
(87) International publication number: WO 2023/030332

(57) **Abstract**

A steroidal compound, a preparation method therefor and an application thereof. On one hand, provided are a compound represented by formula I, a preparation method therefor, and an application thereof in a method for separating and purifying lanosterol; on the other hand, provided is the method for separating and purifying lanosterol. The method is simple to operate, stable in process, high in productivity and low in cost, and the obtained lanosterol has high purity and can meet medical application thereof.

## Description

The present application claims the priority to Chinese Patent Application No. 2021110137424 filed on Aug 31, 2021, which is incorporated herein by reference in its entirety.

### TECHNICAL FIELD

The present disclosure relates to the field of separation and purification of organic compounds, and in particular, to a steroid compound, a preparation method therefor, and application thereof.

### BACKGROUND

Lanosterol is a tetracyclic triterpenoid and is an intermediate for the biosynthesis of cholesterol. Currently, lanosterol is mainly obtained by separating and extracting from crude products of lanosterol. However, crude products of lanosterol are mixtures of tetracyclic triterpenoids isolated from lanolin by crystallization, which typically contain about 60% of lanosterol (CAS No.: 79-63-0) and about 30% of dihydrolanosterol (CAS No.: 79-62-9), as well as other impurities, such as cholesterol (CAS No.: 57-88-5), and the like. It is very difficult to separate lanosterol and dihydrolanosterol due to their similar polarities.

Although CN101691391 reported the preparation of lanosterol by high-performance liquid chromatography with a purity of up to 97%, only 250 mg of lanosterol was prepared, which is cost-inefficient and unsuitable for industrial mass production. At present, no purification process for mass production and preparation may produce lanosterol with a purity higher than 90%, making it difficult to meet the demand of pharmaceutical applications.

In addition, the separation of lanosterol from other impurity compounds is also very challenging, and it is almost impossible to completely separate lanosterol by column chromatography and recrystallization. For example, it has been found that after the recrystallization of lanosterol mixed with other impurities, the proportion of impurities in the crystal of lanosterol precipitated does not change significantly from that before the crystallization.

Therefore, for better investigation and application of lanosterol in pharmaceuticals, there's an urgent need for a method for separating and purifying lanosterol.

### SUMMARY

In order to solve one of the technical problems in the prior art, the present disclosure provides a steroid compound, a preparation method therefor, and application thereof. The present disclosure further provides a method for purifying lanosterol. The method can separate and purify lanosterol from a crude product of lanosterol, and features ease to operate, stable processes, high yield, and low cost. The obtained lanosterol has high purity and can meet the requirements of pharmaceutical applications.

In one aspect of the present disclosure, a compound of formula I is provided, 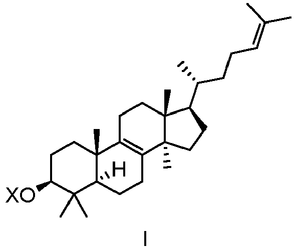 wherein X is 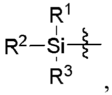 and R¹, R², and R³ are each independently C₁₋₄ alkyl, C₁₋₄ alkoxy, 5- to 9-membered heteroaryl, or C₆₋₁₀ aryl, with the heteroatom in the 5- to 9-membered heteroaryl being selected from N, O, or S, and the number of the heteroatom in the 5- to 9-membered heteroaryl being 1, 2, or 3.

According to some embodiments of the present disclosure, X is TMS, TES, TBS, TBDPS, TIPS, DMIPS, TBDMS, or MDIPS.

In another aspect of the present disclosure, a preparation method for the compound of formula I is provided, comprising: reacting lanosterol with a hydroxyl-protecting agent to give the compound of formula I.

According to some embodiments of the present disclosure, the hydroxyl-protecting agent is a silyl ether-based protecting agent; according to some embodiments of the present disclosure, the hydroxyl-protecting agent is selected from trimethylchlorosilane, triethylchlorosilane, tri-*tert*-butylchlorosilane, *tert*-butyldiphenylchlorosilane, triisopropylchlorosilane, dimethylisopropylchlorosilane, *tert-*butyldimethylchlorosilane, methyldiisopropylchlorosilane, triisopropylchlorosilane, and *tert*-butyldimethylsilyl trifluoromethanesulfonate.

According to some embodiments of the present disclosure, the reaction of lanosterol with the hydroxyl-protecting agent may be conducted in the presence of an organic solvent. According to some other embodiments of the present disclosure, the organic solvent is *N*,*N-*dimethylformamide or dichloromethane.

According to some embodiments of the present disclosure, the reaction of lanosterol with the hydroxyl-protecting agent is conducted in the presence of a deacid reagent; according to some other embodiments of the present disclosure, the deacid reagent is an organic base or an inorganic base; according to still some other embodiments of the present disclosure, the deacid reagent is pyridine, imidazole, diisopropylamine, triethylamine, triethanolamine, potassium carbonate, or sodium carbonate.

According to some embodiments of the present disclosure, the reaction of lanosterol with the hydroxyl-protecting agent may be conducted at a temperature of 25-120 °C. According to some embodiments of the present disclosure, the reaction of the crude product of lanosterol with the hydroxyl-protecting agent may be conducted at a temperature of 60-85 °C.

In another aspect of the present disclosure, a separation and purification method for the compound of formula I is provided, comprising: separating and purifying a raw material A by column chromatography to give the compound of formula I, wherein the raw material A comprises the compound of formula I and a compound of formula I';

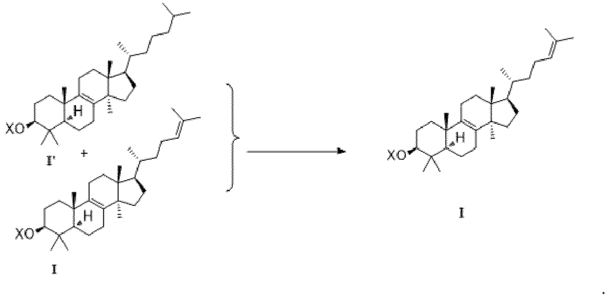

According to some embodiments of the present disclosure, in the raw material A, the mass percentage of the compound I is 45%-85%; according to some embodiments of the present disclosure, in the raw material A, the mass percentage of the compound I is 55%-70%; according to some embodiments of the present disclosure, in the raw material A, the mass percentage of the compound I is 60%-70%.

According to some embodiments of the present disclosure, the column chromatography is silica gel column chromatography.

According to some embodiments of the present disclosure, the specification of the silica gel selected for the silica gel column chromatography is 100-200 mesh, 200-300 mesh, or 300-400 mesh.

According to some embodiments of the present disclosure, the eluent used for the silica gel column chromatography is one or more of petroleum ether, *n*-heptane, *n*-hexane, dichloromethane, and ethyl acetate. According to some embodiments of the present disclosure, aqueous ammonia is added to the eluent used for the silica gel column chromatography. According to some embodiments of the present disclosure, the eluent used for the silica gel column chromatography may be a mixture of *n*-heptane and ethyl acetate, a mixture of *n*-hexane and ethyl acetate, or a mixture of *n*-heptane, ethyl acetate, and aqueous ammonia. According to some embodiments of the present disclosure, the eluent used for the silica gel column chromatography may be a mixture of *n*-heptane and ethyl acetate, wherein the volume ratio of *n*-heptane to ethyl acetate is (90-100):(10-0), e.g., 100:1. According to some embodiments of the present disclosure, the eluent used for the silica gel column chromatography may be a mixture of *n*-hexane and ethyl acetate, wherein the volume ratio of *n*-hexane to ethyl acetate is (90-100):(10-0), e.g., 100:1.

According to some embodiments of the present disclosure, the raw material A is mixed with silica gel of 100-200 mesh before the silica gel column chromatography described above.

According to some embodiments of the present disclosure, the silica gel column chromatography may be conducted 1 or more times, e.g., 1, 2, 3, 4, or 5 times.

According to some embodiments of the present disclosure, the separation and purification method for the compound of formula I further comprises: reacting a crude product of lanosterol with a hydroxyl-protecting agent to give the raw material A;

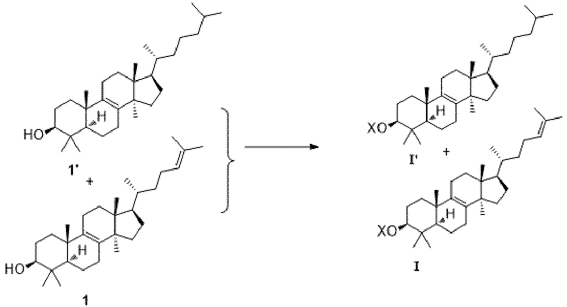

According to some embodiments of the present disclosure, the hydroxyl-protecting agent is a silyl ether-based protecting agent, e.g., selected from trimethylchlorosilane, triethylchlorosilane, tri-*tert*-butylchlorosilane, *tert*-butyldiphenylchlorosilane, triisopropylchlorosilane, dimethylisopropylchlorosilane, *tert*-butyldimethylchlorosilane, methyldiisopropylchlorosilane, triisopropylchlorosilane, and *tert*-butyldimethylsilyl trifluoromethanesulfonate.

According to some embodiments of the present disclosure, the reaction of the crude product of lanosterol with the hydroxyl-protecting agent is conducted in the presence of an organic solvent. According to some embodiments of the present disclosure, the organic solvent is *N*,*N-*dimethylformamide or dichloromethane.

According to some embodiments of the present disclosure, the reaction of the crude product of lanosterol with the hydroxyl-protecting agent is conducted in the presence of a deacid reagent. According to some embodiments of the present disclosure, the deacid reagent is an organic base or an inorganic base; according to some embodiments of the present disclosure, the deacid reagent is pyridine, imidazole, diisopropylamine, triethylamine, triethanolamine, potassium carbonate, or sodium carbonate.

According to some embodiments of the present disclosure, the reaction of the crude product of lanosterol with the hydroxyl-protecting agent is conducted at a temperature of 25-120 °C; according to some embodiments of the present disclosure, the reaction of the crude product of lanosterol with the hydroxyl-protecting agent is conducted at a temperature of 60-85 °C.

In another aspect of the present disclosure, use of the compound of formula I in separating and purifying lanosterol is provided.

According to some embodiments of the present disclosure, the use comprises: conducting hydroxyl deprotection to the compound of formula I to give lanosterol;

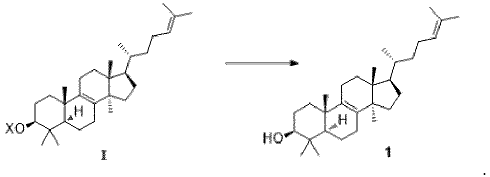

According to some embodiments of the present disclosure, the hydroxyl deprotection is conducted in the presence of one or more of acetic acid, tetraalkylammonium fluoride, trifluoroacetic acid, or hydrochloric acid. According to some embodiments of the present disclosure, the hydroxyl deprotection is conducted in the presence of tetrabutylammonium fluoride.

According to some embodiments of the present disclosure, the use further comprises: separating and purifying a raw material A by column chromatography to give the compound of formula I, wherein the raw material A comprises the compound of formula I and a compound of formula I'; 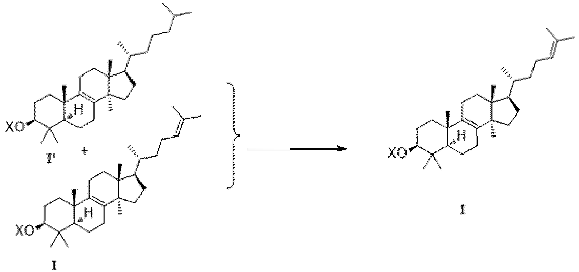 wherein X is 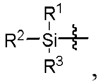 and R¹, R², and R³ are each independently C₁₋₄ alkyl, C₁₋₄ alkoxy, 5- to 9-membered heteroaryl, or C₆₋₁₀ aryl, with the heteroatom in the 5- to 9-membered heteroaryl being selected from N, O, or S, and the number of the heteroatom in the 5- to 9-membered heteroaryl being 1, 2, or 3.

According to some embodiments of the present disclosure, X is TMS, TES, TBS, TBDPS, TIPS, DMIPS, TBDMS, or MDIPS.

According to some embodiments of the present disclosure, in the raw material A, the mass percentage of the compound I is 45%-85%; according to some embodiments of the present disclosure, in the raw material A, the mass percentage of the compound I is 55%-70%; according to some embodiments of the present disclosure, in the raw material A, the mass percentage of the compound I is 60%-70%.

According to some embodiments of the present disclosure, the column chromatography is silica gel column chromatography.

According to some embodiments of the present disclosure, the specification of the silica gel selected for the silica gel column chromatography is 100-200 mesh, 200-300 mesh, or 300-400 mesh.

According to some embodiments of the present disclosure, the eluent used for the silica gel column chromatography is one or more of petroleum ether, n-heptane, n-hexane, dichloromethane, and ethyl acetate. According to some embodiments of the present disclosure, aqueous ammonia is added to the eluent used for the silica gel column chromatography. According to some embodiments of the present disclosure, the eluent used for the silica gel column chromatography may be a mixture of *n*-heptane and ethyl acetate, a mixture of *n*-hexane and ethyl acetate, or a mixture of *n*-heptane, ethyl acetate, and aqueous ammonia. According to some embodiments of the present disclosure, the eluent used for the silica gel column chromatography may be a mixture of n-heptane and ethyl acetate, wherein the volume ratio of *n*-heptane to ethyl acetate is (90-100):(10-0), e.g., 100:1. According to some embodiments of the present disclosure, the eluent used for the silica gel column chromatography may be a mixture of *n*-hexane and ethyl acetate, wherein the volume ratio of *n*-hexane to ethyl acetate is (90-100):(10-0), e.g., 100:1.

According to some embodiments of the present disclosure, the raw material A is mixed with silica gel of 100-200 mesh before the silica gel column chromatography described above.

According to some embodiments of the present disclosure, the silica gel column chromatography may be conducted 1 or more times, e.g., 1, 2, 3, 4, or 5 times.

According to some embodiments of the present disclosure, the use further comprises: reacting a crude product of lanosterol with a hydroxyl-protecting agent to give the raw material A;

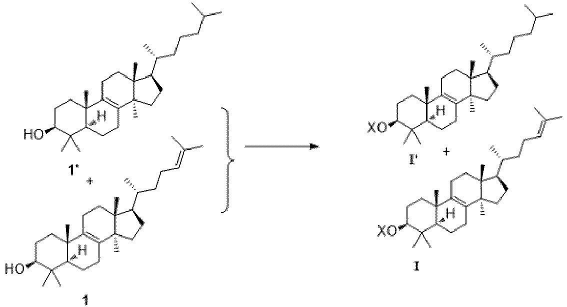

According to some embodiments of the present disclosure, the hydroxyl-protecting agent is a silyl ether-based protecting agent, e.g., selected from trimethylchlorosilane, triethylchlorosilane, tri-*tert*-butylchlorosilane, *tert*-butyldiphenylchlorosilane, triisopropylchlorosilane, dimethylisopropylchlorosilane, *tert*-butyldimethylchlorosilane, methyldiisopropylchlorosilane, triisopropylchlorosilane, and *tert*-butyldimethylsilyl trifluoromethanesulfonate.

According to some embodiments of the present disclosure, the reaction of the crude product of lanosterol with the hydroxyl-protecting agent is conducted in the presence of an organic solvent. According to some embodiments of the present disclosure, the organic solvent is *N*,*N-*dimethylformamide or dichloromethane.

According to some embodiments of the present disclosure, the reaction of the crude product of lanosterol with the hydroxyl-protecting agent is conducted in the presence of a deacid reagent. According to some embodiments of the present disclosure, the deacid reagent is an organic base or an inorganic base; according to some embodiments of the present disclosure, the deacid reagent is pyridine, imidazole, diisopropylamine, triethylamine, triethanolamine, potassium carbonate, or sodium carbonate.

According to some embodiments of the present disclosure, the reaction of the crude product of lanosterol with the hydroxyl-protecting agent is conducted at a temperature of 25-120 °C; according to some embodiments of the present disclosure, the reaction of the crude product of lanosterol with the hydroxyl-protecting agent is conducted at a temperature of 60-85 °C.

In another aspect of the present disclosure, a method for refining a compound of formula I is provided, comprising: recrystallizing a raw material A to give the compound of formula I, wherein the raw material A comprises the compound of formula I and a compound of formula I'; 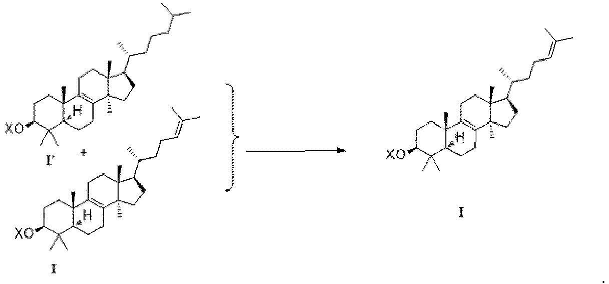 wherein X is as defined above, and the solvent for the recrystallization is selected from:
1) a mixture of ethyl acetate and isopropanol, or 2) isopropyl acetate.

According to some embodiments of the present disclosure, in the mixture of ethyl acetate and isopropanol, the volume ratio of ethyl acetate to isopropanol is 1:(0.5-10), preferably 1:(1-10), e.g., 1:(0.5-2).

According to some embodiments of the present disclosure, the mass percentage of the compound of formula I in the raw material A is 75% or greater, preferably 85% or greater, and more preferably 90% or greater.

According to some embodiments of the present disclosure, the recrystallization comprises dissolving the raw material A in the solvent, heating until the raw material A is completely dissolved, and cooling the system to room temperature.

In another aspect of the present disclosure, a composition Y is provided, comprising a compound of formula I and a compound of formula I', 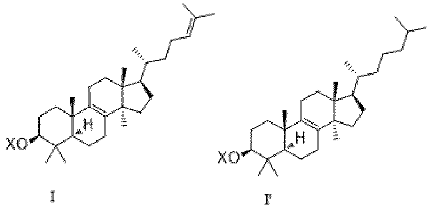 wherein X is 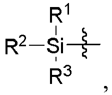 and R¹, R², and R³ are each independently C₁₋₄ alkyl, C₁₋₄ alkoxy, 5- to 9-membered heteroaryl, or C₆₋₁₀ aryl, with the heteroatom in the 5- to 9-membered heteroaryl being selected from N, O, or S, and the number of the heteroatom in the 5- to 9-membered heteroaryl being 1, 2, or 3.

According to some embodiments of the present disclosure, X is TMS, TES, TBS, TBDPS, TIPS, DMIPS, TBDMS, or MDIPS.

According to some embodiments of the present disclosure, in the composition Y, the mass percentage of the compound of formula I is 55%-95%, e.g., 56%, 57%, 58%, 59%, 60%, 61%, 65%, 70%, 75%, 80%, 81%, 82%, 83%, 84%, 85%, 86%, 87%, 88%, 89%, 90%, 91%, 92%, 93%, or 94%.

In another aspect of the present disclosure, a method for purifying lanosterol is provided, comprising:
1) reacting a crude product of lanosterol with a hydroxyl-protecting agent to give the raw material A, the raw material A comprising a compound of formula I and a compound of formula I';
2) separating the raw material A by column chromatography to give the compound of formula I; and
3) conducting hydroxyl deprotection to the compound of formula I to give purified lanosterol;

wherein the crude product of lanosterol comprises lanosterol and dihydrolanosterol;
the hydroxyl-protecting agent is a silyl ether-based protecting agent.

According to some embodiments of the present disclosure, the silyl ether-based protecting agent is selected from trimethylchlorosilane, triethylchlorosilane, *tri-tert-*butylchlorosilane, *tert*-butyldiphenylchlorosilane, triisopropylchlorosilane, dimethylisopropylchlorosilane, *tert*-butyldimethylchlorosilane, methyldiisopropylchlorosilane, triisopropylchlorosilane, and *tert*-butyldimethylsilyl trifluoromethanesulfonate;
wherein, in the method for purifying lanosterol, the raw material A, the compound of formula I, and the reaction conditions and the procedures of each step are as described in any of the preceding embodiments.

In order to purify a crude product of lanosterol (the raw material), the crude product of lanosterol is subj ected to hydroxyl protection by silyl ether. The lanosterol with hydroxyl protected by silyl ether and the dihydrolanosterol with hydroxyl protected by silyl ether can be separated by silica gel column chromatography due to their difference in polarity, so as to give pure lanosterol with hydroxyl protected by silyl ether. The silyl ether-based protecting group is then removed to give pure lanosterol. Moreover, according to the present disclosure, it is found that the lanosterol with hydroxyl protected by silyl ether has good crystallization properties. If the recrystallization is performed before removing the protecting group, some impurities can be retained in the mother liquor. The hydroxyl-protecting group is removed to give the purified lanosterol. The obtained lanosterol not only meets the commercial demand of large-scale preparation, but also meets the demand of pharmaceutical applications. It has been demonstrated by a large number of experiments that other non-silyl ether-based hydroxyl-protecting groups, such as alkyl ethers, carboxylic acid esters, and amino acid esters, areunable to separate lanosterol from dihydrolanosterol.

The following examples are provided to facilitate the understanding of the present disclosure. However, it will be appreciated that such examples are illustrative only and should not be construed as limiting the present disclosure in any way. The actual protection scope of the present disclosure is illustrated in the claims. It will be appreciated that any modifications and changes may be made without departing from the spirit of the present disclosure.

### DETAILED DESCRIPTION

In order to make the objects, embodiments, and advantages of the present disclosure more apparent, the present disclosure is further described in detail with reference to the following examples. The specific examples described herein are merely illustrative of the present disclosure and do not constitute any limitation thereon. Moreover, in the following description, descriptions of well-known structures and techniques are omitted so as to avoid unnecessary obscuration of concepts in the present disclosure. Such structures and techniques are also described in numerous publications.

Unless defined otherwise, all technical and scientific terms used herein have the same meaning as commonly used in the art to which the present disclosure belongs. For the purpose of illustrating the present disclosure, the following definitions will apply, and, where appropriate, terms used in the singular form will also include the plural form and vice versa.

As used herein, the terms "a" and "an" include plural references unless otherwise stated. For example, reference to "a cell" includes a plurality of such cells and equivalents thereof known to those skilled in the art, and so forth.

The term "about" as used herein means a range of ±20% of the value following it. In some embodiments, the term "about" means a range of ±10% of the value following it. In some embodiments, the term "about" means a range of ±5% of the value following it.

The solvents used herein are commercially available. The following abbreviations are used herein:
TIPS: triisopropylsilane
TIPSCI: triisopropylchlorosilane
DMIPS: dimethylisopropylsilane
DMIPSCl: dimethylisopropylchlorosilane
TES: triethylsilane
TESCl: triethylchlorosilane
TMS: trimethylsilane
TBDPS: *tert*-butyldiphenylsilane
TBDPSCl: *tert*-butyldiphenylchlorosilane
TBS: *tert*-butyldimethylsilane
TBSCl: *tert*-butyldimethylchlorosilane
TMSCI: trimethylchlorosilane
TLC: thin-layer chromatography
TBAF: tetrabutylammonium fluoride
THF: tetrahydrofuran
DCM: dichloromethane
DMF: dimethylformamide
eq: equivalent

Compounds are named either manually or by ChemDraw^{®} software, and supplier's catalog names are given for commercially available compounds.

Lanosterol material (crude) (supplied by Shandong Jun Rui Co., Ltd. or Spectrum Chemical Manufacturing Corp-China)

### Examples

In the column chromatography, the conditions used to track the elution of the target intermediate in the eluent by thin-layer chromatography (TLC) were: petroleum ether: ethyl acetate = 5:1, color development: phosphomolybdic acid.

Based on experience and nuclear magnetic resonance analysis, the TLC results of the collected eluate containing the target intermediate were divided into three types: those basically free of impurity spots (defined as high-purity products with a ¹H NMR characteristic peak integral ratio or HPLC purity of 85%-90%), those with impurity spots but significant improvement in purity (defined as relatively high-purity products with a ¹H NMR characteristic peak integral ratio or HPLC purity of 70%-85%), and those with impurity spots, no significant improvement in purity, and a higher concentration of the target intermediate in the eluate (defined as low-purity products with a ¹H NMR characteristic peak integral ratio or HPLC purity generally less than 70%). The improvement in purity was relative to the purity of the sample of the current column chromatography.

### Example 1

### 1.1 Protection of hydroxyl

500 g of a commercial lanosterol material (about 60% lanosterol purity) was aliquoted into two replicates of 250 g each. Each replicate was subjected to the following processing.

At 15 °C, imidazole (87.8 g, 1.29 mol) and TBSCl (132.5 g, 879 mmol) were added to a solution of a lanosterol material (250 g) in *N*,*N*-dimethylformamide (2500 mL). The mixture was stirred at 85 °C for 6 h. The mixture was then cooled to 15 °C and extracted with petroleum ether (2500 × 2 mL). The petroleum ether phase was washed sequentially with a saturated NaCl solution (2000 mL) and water (2000 mL).

348 g of intermediate 1a and 350 g of intermediate 1b were acquired.

According to the general knowledge in the art, the difference in purity between the intermediates 1a and 1b and the lanosterol material before and after the hydroxyl protection reaction is negligible.

### 1.2 Column chromatography

348 g of intermediate 1a was mixed with 1000 g of silica gel of 100-200 mesh, and then subjected by column chromatography (1000 mm × 230 mm) with 10 kg of silica gel of 200-300 mesh. The column was eluted using a mixture ofn-heptane and ethyl acetate (100:1). The elution process was tracked by TLC. An eluate containing a relatively high-purity product was collected, evaporated at a reduced pressure, and dried to give 125 g of intermediate 1c (66% yield).

350 g of intermediate 1b was mixed with 1000 g of silica gel of 100-200 mesh, and then subjected by column chromatography (1000 mm × 230 mm) with 10 kg of silica gel of 200-300 mesh. The column was eluted using a mixture of *n*-heptane and ethyl acetate (100:1). The elution process was tracked by TLC. An eluate containing a high-purity product was collected and dried to give 121 g of intermediate 1d (64% yield).

Intermediates 1c and 1d were combined and mixed with 740 g of silica gel of 100-200 mesh, and then subjected by column chromatography (1000 mm × 230 mm) with 7.4 kg of silica gel of 200-300 mesh. The column was eluted using a mixture of *n*-heptane and ethyl acetate (100:1). The elution process was tracked by TLC. An eluate containing a high-purity product was collected, evaporated at a reduced pressure, and dried to give 179 g of intermediate 1e (47% yield).

179 g of intermediate 1e was mixed with 540 g of silica gel of 100-200 mesh, and then subjected by column chromatography (1000 mm × 230 mm) with 5.4 kg of silica gel of 200-300 mesh. The column was eluted using a mixture of *n*-hexane and ethyl acetate (100:1). The elution process was tracked by TLC. An eluate containing a high-purity product was collected and dried to give 161 g of intermediate 1f (42% yield). Faint impurity spots were observed by the TLC monitoring of the intermediate 1f, and the ¹H NMR characteristic peak integral showed that TBS-lanosterol accounted for about 83% of the total sterols.

161 g of intermediate 1f was mixed with 480 g of silica gel of 100-200 mesh, and then subjected by column chromatography (1000 mm × 230 mm) with 5.0 kg of silica gel of 200-300 mesh. The column was eluted using a mixture of *n*-hexane and ethyl acetate (100:1). The elution process was tracked by TLC. An eluate containing a high-purity product was collected, evaporated at a reduced pressure, and dried to give 147 g of intermediate 1h. The overall yield of the 4 purifications was about 39%. Unremarkable impurity spots were observed in the TLC monitoring of the intermediate 1h, and the ¹H NMR characteristic peak integral showed that TBS-lanosterol accounted for about 91% of the total sterols.

¹H NMR (400 MHz, CDCl₃) δ = 5.07 (br t, *J* = 7.2 Hz, 1H), 3.17 (dd, *J* = 4.6, 11.2 Hz, 1H), 2.05-1.35 (m, 24H), 1.29 (br s, 5H), 1.18-0.97 (m, 5H), 0.95 (s, 3H), 0.90-0.88 (m, 4H), 0.84 (s, 9H), 0.74 (s, 3H), 0.66 (s, 3H), 0.00 (d, *J* = 2.5 Hz, 7H) ppm.

### 1.3 Deprotection of hydroxyl

49.2 g of the intermediate 1h was dissolved in 250 mL of anhydrous tetrahydrofuran. At 15 °C, 110 mL of a TBAF solution (1.0 M) was added, and the mixture was heated to reflux for 16 h. The reaction was monitored by TLC. After the reaction was completed, the reaction solution was directly concentrated by rotary evaporation at a reduced pressure to give a residue. 600 mL of methanol was added to the residue, and the mixture was refluxed for 4 h to give a white suspension. The suspension was stirred at 60 °C for 24 h, and then cooled to 15 °C. The mixture was filtered to give a filter cake. The filter cake was washed with 300 mL of methanol and then dried at a reduced pressure to give 38.3 g of final product (98.4% yield; 91% lanosterol purity by HPLC).

¹H NMR (400 MHz, CDCl₃) δ 5.12 (br t, *J* = 7.15 Hz, 1H), 3.26 (dd, *J* = 4.64, 11.42 Hz, 1H), 1.73-2.13 (m, 10H), 1.71 (s, 3H), 1.64-1.68 (m, 1H), 1.63 (s, 3H), 1.05-1.59 (m, 12H), 1.02 (s, 3H), 1.00 (s, 3H), 0.93 (d, *J* = 6.27 Hz, 3H), 0.90 (s, 3H), 0.83 (s, 3H), 0.71 (s, 3H) ppm.

### Example 2

### 2.1 Protection of hydroxyl

At 15 °C, imidazole (87.8 g, 1.29 mol) and TESCl (132.5 g, 879 mmol) were added to a solution of a commercial lanosterol material (250 g, about 60% lanosterol purity) in *N,N-*dimethylformamide (2500 mL). The mixture was stirred at 80 °C for 3 h. The reaction was monitored by TLC. After the reaction was completed, the reaction mixture was cooled to 25 °C, and 600 mL of methanol and 300 mL of water were added. The mixture was stirred and filtered to give a filter cake. The filter cake was washed with 300 mL of methanol, and then dried at a reduced pressure to give 341 g of intermediate 2a.

### 2.2 Column chromatography

341 g of intermediate 2a was mixed with 1000 g of silica gel of 100-200 mesh, and then subjected by column chromatography (1000 mm × 230 mm) with 10 kg of silica gel of 200-300 mesh. The column was eluted using a mixture ofn-heptane and ethyl acetate (100:1). The elution process was tracked by TLC. An eluate containing a high-purity product was collected, evaporated at a reduced pressure, and dried to give 168 g of intermediate 2b (88% yield) (faint impurity spots were still observed in TLC).

168 g of intermediate 2b was mixed with 540 g of silica gel of 100-200 mesh, and then subjected by column chromatography (1000 mm × 180 mm) with 5.4 kg of silica gel of 200-300 mesh. The column was eluted using a mixture of *n*-heptane and ethyl acetate (100:1). The elution process was tracked by TLC. An eluate basically free of impurity spots was collected, evaporated at a reduced pressure, and dried to give 117 g of intermediate 2c (about 62% yield). The ¹H NMR characteristic peak integral showed that TES-lanosterol accounted for about 86% of the total sterols.

### 2.3 Deprotection of hydroxyl

At 25 °C, 27 g of intermediate 2c was dissolved in anhydrous THF (150 mL) and TBAF (55 mL, 0.055 mol). The mixture was incubated for 30 min for reaction. Then, the reaction mixture was warmed to 60 °C, refluxed, and stirred for 4 h. The reaction completion was monitored by TLC. 80 mL of water and 160 mL of methanol were added, and the mixture was stirred for 1 h. A solid was precipitated. The mixture was filtered to give a filter cake. The filter cake was washed with a small amount of water and methanol, and then dried at a reduced pressure to give 18.5 g of the product (white solid, 87% yield; about 89% purity lanosterol by HPLC).

### Example 3

### 3.1 Protection of hydroxyl

At 15 °C, imidazole (87.8 g, 1.29 mol) and TESCl (132.5 g, 879 mmol) were added to a solution of a commercial lanosterol material (250 g, about 60% lanosterol purity) in *N,N-*dimethylformamide (2500 mL). The mixture was stirred at 80 °C for 3 h. The reaction was monitored by TLC. After the reaction was completed, the reaction mixture was cooled to 25 °C, and 600 mL of methanol and 300 mL of water were added. The mixture was stirred and filtered to give a filter cake. The filter cake was washed with 300 mL of methanol, and then dried at a reduced pressure to give 355 g of intermediate 3a.

### 3.2 Column chromatography

355 g of intermediate 3a was mixed with 1000 g of silica gel of 100-200 mesh, and then subjected by column chromatography (1000 mm × 230 mm) with 10 kg of silica gel of 200-300 mesh. The column was eluted using a mixture of *n*-heptane and ethyl acetate (100:1). The elution process was tracked by TLC. Eluates containing a high-purity product and a relatively high-purity product were collected, evaporated at a reduced pressure, and dried to give 71 g of intermediate 3b with a purity of about 87%-89% and 96 g of intermediate 3c with a purity of about 70%-80%, respectively.

96 g of intermediate 3c was mixed with 300 g of silica gel of 100-200 mesh, and then subjected by column chromatography (1000 mm × 180 mm) with 3 kg of silica gel of 200-300 mesh. The column was eluted using a mixture of *n*-heptane and ethyl acetate (100:1). The elution process was tracked by TLC. An eluate containing a relatively high-purity product was collected, evaporated at a reduced pressure, and dried to give 44 g of intermediate 3d.

A total of 115 g of intermediates 3b and 3d (designated as intermediate 3e) was combined (61% yield). The ¹H NMR characteristic peak integral showed that TES-lanosterol accounted for about 90% of the total sterols.

### 3.3 Deprotection of hydroxyl

At 25 °C, intermediate 3e (22 g, 0.04 mol) was dissolved in anhydrous THF (120 mL). TBAF (45 mL, 0.045 mol) was added to the reaction solution. The mixture was let stand for 30 min. Then, the reaction mixture was warmed to 60 °C, refluxed, and stirred for 4 h. The reaction was monitored by TLC. After the reaction was completed, 60 mL of water and 120 mL of methanol were added, and the mixture was stirred for 1 h. A solid was precipitated. The solid was filtered to give a filter cake. The filter cake was washed with 60 mL of water and then washed with 100 mL of methanol. The mixture was filtered to give a filter cake. The filter cake was refluxed in 200 mL of methanol for 3 h, and then cooled to precipitate a solid. Again, the mixture was filtered to give 15.2 g of a white solid (88% yield; about 92% lanosterol purity by HPLC).

### Example 4

### 4.1 Recrystallization

50 g of the intermediate 3e obtained in Example 3 was added to a 750-mL mixture of ethyl acetate and isopropanol (volume ratio = 1:1). The mixture was warmed to 90 °C and refluxed for 2 h until the solids were completely dissolved. The stirring was stopped and the mixture was slowly cooled. A solid was precipitated. The mixture was filtered to give a filter cake. The filter cake was washed and dried to give 40 g of intermediate 4a (80% yield). The ¹H NMR characteristic peak integral showed that TES-lanosterol accounted for about 93% of the total sterols.

¹H NMR (400 MHz, CDCl₃) δ 0.56-0.66 (m, 6H), 0.70-0.91 (m, 9H), 0.92-1.07 (m, 19H), 1.12-1.77 (m, 21H), 1.82-2.12 (m, 7H), 3.25 (dd, *J* = 11.29, 4.52 Hz, 1H), 5.12 (br t, *J* = 7.03 Hz, 1H).

### 4.2 Deprotection

At 25 °C, intermediate 4a (20 g, 0.037 mol) was dissolved in anhydrous THF (120 mL). TBAF (1 M, 1.5 eq) was added to the reaction solution. The reaction mixture was let stand for 30 min. The reaction mixture was warmed to 60 °C, refluxed, and stirred for 4 h. The reaction completion was monitored by TLC. 60 mL of water and 120 mL of methanol were added, and the mixture was stirred for 1 h. A solid was precipitated. The solid was filtered to give a filter cake. The filter cake was washed sequentially with a small amount of water and methanol. The mixture was filtered to give a filter cake. The filter cake was refluxed for 3 h in 200 mL of methanol, and then cooled to precipitate a solid. The solid was filtered to give 13 g of a white solid. The yield was about 85%, and the lanosterol purity was >99% as measured by HPLC.

### Example 5

### 5.1: Protection of hydroxyl

At 15 °C, imidazole (87.75 g, 1.29 mol) and TMSCl (95.47 g, 879 mmol) were added to a solution of a commercial lanosterol material (250 g, about 60% lanosterol purity) in *N,N-*dimethylformamide (2500 mL). The mixture was stirred at 70 °C for 5 h. After the reaction was completed, the reaction solution was cooled to 15 °C and extracted with petroleum ether (2500 × 2 mL). The petroleum ether phase was washed with a saturated NaCl solution (2000 mL) and water (2000 mL) to give 287 g of intermediate 5a.

### 5.2: Column chromatography

287 g of intermediate 5a was mixed with 850 g of silica gel of 100-200 mesh, and then subjected by column chromatography (1000 mm × 230 mm) with 8.5 kg of silica gel of 200-300 mesh. The column was eluted using a mixture of n-heptane, ethyl acetate, and aqueous ammonia (100:1:0.05). The elution process was tracked by TLC. An eluate was collected, evaporated at a reduced pressure, and dried to give 53 g of product 5b. The yield of TMS-lanosterol intermediate was about 30% with a purity of about 80% as determined by ¹H NMR. TLC showed faint impurity spots.

### Example 6

### Investigation of protecting agents and conditions

At 15 °C, imidazole (3.9 g) and triethylchlorosilane (1-2 eq.) were added to a solution of a commercial lanosterol material (10 g, 0.023 mol, about 60% purity) in DMF (100 mL). The reaction completion was monitored by TLC. The following post-treatments were performed for reactions with relatively complete conversion: The reaction mixture was cooled to room temperature and extracted with petroleum ether (75 × 2 mL). The petroleum ether phase obtained was washed with a saturated NaCl solution (75 mL) and water (750 mL), and then dried to give a corresponding crude product. The product was purified by column chromatography.

The solvent, protecting agent, temperature, and reaction time in the reaction described above were changed, and the corresponding test conditions and results are shown in the following table:

| **No.** | **Hydroxyl-protecting agent** | **Solvent** | **Temperature, °C** | **Time** | **TLC results** | **Column chromatography results** |
|---|---|---|---|---|---|---|
| **1** | Benzoic acid | DCM | 60 | 24 h | Irrecognizable impurity spots | None |
| **2** | p-Nitrobenzoic acid | DCM | 60 | 24 h | Irrecognizable impurity spots | None |
| **3** | 2-Picolinic acid | DCM | 60 | 24 h | Irrecognizable impurity spots | None |
| **4** | Acetic acid | DCM | 60 | 24 h | Irrecognizable impurity spots | None |
| **5** | Glycine | DCM | 60 | 24 h | Incomplete reaction with unstable resultant ester | None |
| **6** | TBSCl (1 eq) | DMF | 85 | 12 h | Recognizable impurity spots with a small amount of raw material | None |
| **7** | TBSCl (1.5 eq) | DMF | 85 | 6 h | Complete conversion with recognizable impurity spots | About 37% yield and about 86% purity |
| **8** | TBSCl (1.5 eq) | DMF | 120 | 4 h | Complete conversion with recognizable impurity spots | About 34% yield and about 86% purity |
| **9** | TBSCl (1.5 eq) | DMF | 70 | 12 | Basically complete conversion with recognizable impurity spots | About 32% yield and about 85% purity |
| **10** | TBSCl (1.5 eq) | DMF | 40 | 12 h | Incomplete reaction with recognizable impurity spots | None |
| **11** | TESCl (1.5 eq) | DMF | 80 | 4 h | Complete conversion with recognizable impurity spots | About 58% yield and about 87% purity |
| **12** | TESCl (1.1 eq) | DMF | 80 | 6 h | Complete conversion with recognizable impurity spots | About 51% yield and about 86% purity |
| **13^{a}** | TESCl (1.2 eq) | DMF | 80 | 4 h | Complete conversion with recognizable impurity spots | About 59% yield and about 87% purity |

**Note:** a represents the following post-treatment procedures: the reaction system was cooled to room temperature; methanol (25 mL) and water (12.5 mL) were added; the mixture was stirred and filtered to give a filter cake; the filter cake was washed with a small amount of methanol, and then dried at a reduced pressure to give a corresponding crude product; the product was purified by column chromatography. In the post-treatment procedures, the product was directly precipitated and then filtered.

### Example 7

5.41 g of intermediate 2c purified by column chromatography obtained in Example 2 was tested with recrystallization conditions as shown in the following table, wherein the mass-volume ratio of the solute to the solvent was 1 g: 10 mL.

| **No.** | **Solvent (ratio)** | **Other procedures** | **Times** | **Yield** | **Purity** |
|---|---|---|---|---|---|
| 1 | Ethyl acetate: isopropanol (volume ratio = 1:1) | The mixture was refluxed for 2 h until complete dissolution, cooled to room temperature, and let stand overnight to precipitate a solid; the solid was filtered and dried at a reduced pressure | 1 | 84% | 91% |
| 2 | Ethyl acetate:isopropanol (1:1) | The mixture was refluxed for 2 h until complete dissolution, cooled to room temperature, and let stand overnight to precipitate a solid; the solid was filtered and dried at a reduced pressure | 2 | 68% | 91% |
| 3 | Isopropyl acetate | The mixture was refluxed for 2 h until complete dissolution, cooled to room temperature, and let stand overnight to precipitate a solid; the solid was filtered and dried at a reduced pressure | 1 | 80% | 90% |
| 4 | Isopropyl acetate | The mixture was refluxed for 2 h until complete dissolution, cooled to room temperature, and let stand overnight to precipitate a solid; the solid was filtered and dried at a reduced pressure | 2 | 68% | 90% |

The embodiments of the present disclosure are not limited to the specific examples described above, and any technical modifications made according to the embodiments of the present disclosure shall fall within the protection scope of the present disclosure.

## Claims

1. A compound of formula I, 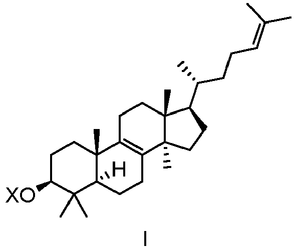 wherein X is 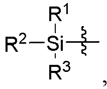 and R¹, R², and R³ are each independently C₁₋₄ alkyl, C₁₋₄ alkoxy, 5- to 9-membered heteroaryl, or C₆₋₁₀ aryl, with the heteroatom in the 5- to 9-membered heteroaryl being selected from N, O, or S, and the number of the heteroatom in the 5- to 9-membered heteroaryl being 1, 2, or 3.

2. The compound according to claim 1, wherein X is TMS, TES, TBS, TBDPS, TIPS, DMIPS, TBDMS, or MDIPS.

3. A preparation method for the compound of formula I according to claim 1, comprising: reacting lanosterol with a hydroxyl-protecting agent to give the compound of formula I.

4. The preparation method according to claim 3, wherein the hydroxyl-protecting agent is a silyl ether-based protecting agent.

5. The preparation method according to claim 3, wherein the hydroxyl-protecting agent is selected from trimethylchlorosilane, triethylchlorosilane, tri-tert-butylchlorosilane, *tert-*butyldiphenylchlorosilane, triisopropylchlorosilane, dimethylisopropylchlorosilane, *tert-*butyldimethylchlorosilane, methyldiisopropylchlorosilane, triisopropylchlorosilane and *tert-*butyldimethylsilyl trifluoromethanesulfonate.

6. A separation and purification method for the compound of formula I according to claim 1 or 2, comprising: separating and purifying a raw material A by column chromatography to give the compound of formula I, wherein the raw material A comprises the compound of formula I and a compound of formula I';

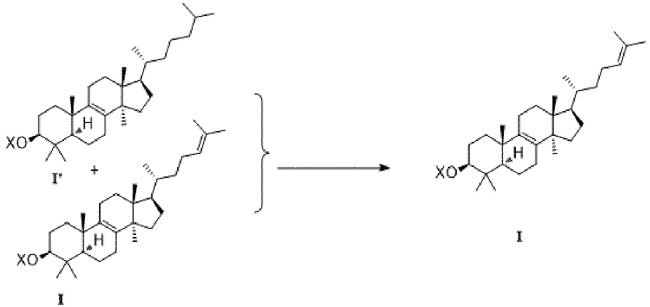

7. The separation and purification method according to claim 6, further comprising: reacting a crude product of lanosterol with a hydroxyl-protecting agent to give the raw material A;

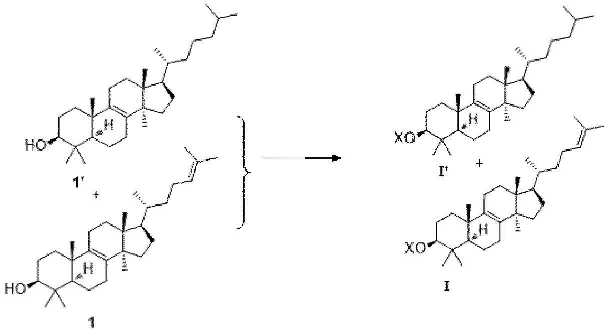

8. The separation and purification method according to claim 7, wherein the hydroxyl-protecting agent is a silyl ether-based protecting agent, e.g., selected from trimethylchlorosilane, triethylchlorosilane, tri-tert-butylchlorosilane, *tert-*butyldiphenylchlorosilane, triisopropylchlorosilane, dimethylisopropylchlorosilane, *tert-*butyldimethylchlorosilane, methyldiisopropylchlorosilane, triisopropylchlorosilane, and *tert-*butyldimethylsilyl trifluoromethanesulfonate.

9. Use of the compound of formula I according to claim 1 or 2 in separating and purifying lanosterol.

10. The use according to claim 9, comprising: conducting hydroxyl deprotection to the compound of formula I to give lanosterol;

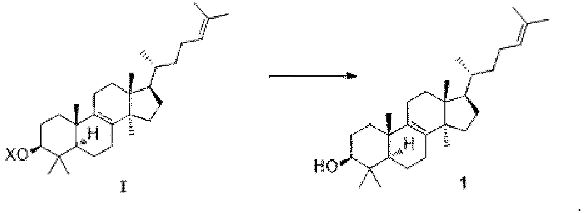

11. The use according to claim 10, further comprising: separating and purifying a raw material A by column chromatography to give the compound of formula I, wherein the raw material A comprises the compound of formula I and a compound of formula I';

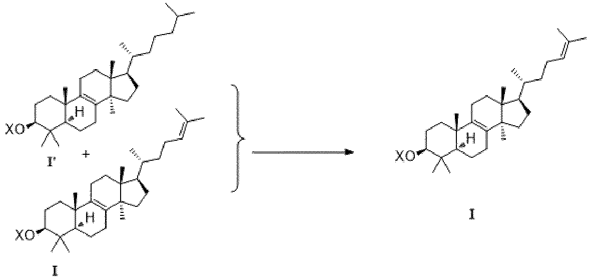

12. The use according to claim 11, further comprising: reacting a crude product of lanosterol with a hydroxyl-protecting agent to give the raw material A;

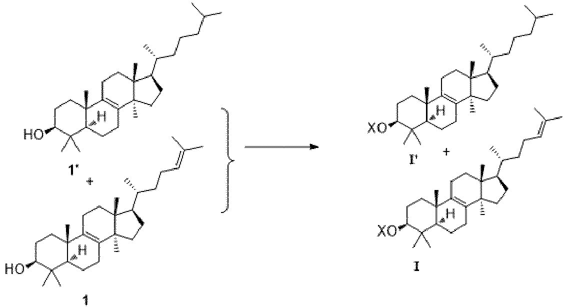

13. A refinement method for a compound of formula I, comprising: recrystallizing a raw material A to give the compound of formula I, wherein the raw material A comprises the compound of formula I and a compound of formula I'; 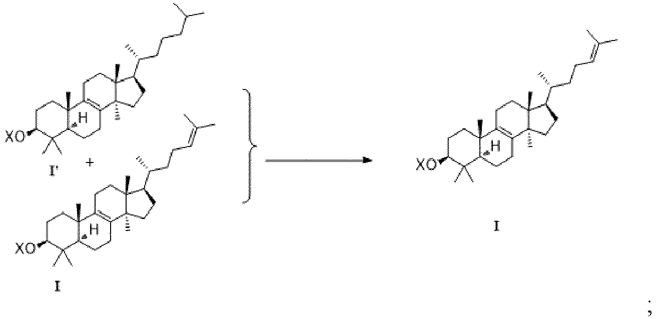 wherein X is as defined in claim 1 or 2, and the solvent for the recrystallization is selected from:
1) a mixture of ethyl acetate and isopropanol, or 2) isopropyl acetate;
preferably, in the mixture of ethyl acetate and isopropanol, the volume ratio of ethyl acetate to isopropanol is 1:(0.5-10), preferably 1:(1-10), e.g., 1:(0.5-2).

14. The refinement method according to claim 13, wherein the mass percentage of the compound of formula I in the raw material A is 75% or greater, preferably 85% or greater, and more preferably 90% or greater.

15. A composition Y, comprising a compound of formula I and a compound of formula I', 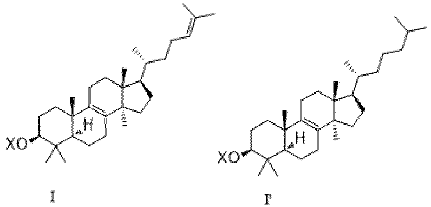 wherein X is 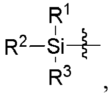 and R¹, R², and R³ are each independently C₁₋₄ alkyl, C₁₋₄ alkoxy, 5- to 9-membered heteroaryl, or C₆₋₁₀ aryl, with the heteroatom in the 5- to 9-membered heteroaryl being selected from N, O, or S, and the number of the heteroatom in the 5- to 9-membered heteroaryl being 1, 2, or 3.

16. The composition Y according to claim 15, wherein X is TMS, TES, TBS, TBDPS, TIPS, DMIPS, TBDMS, or MDIPS.

17. The composition Y according to claim 15 or 16, wherein the mass percentage of the compound of formula I is 55%-95%, e.g., 56%, 57%, 58%, 59%, 60%, 61%, 65%, 70%, 75%, 80%, 81%, 82%, 83%, 84%, 85%, 86%, 87%, 88%, 89%, 90%, 91%, 92%, 93%, or 94%.

18. A method for purifying lanosterol, comprising:
1) reacting a crude product of lanosterol with a hydroxyl-protecting agent to give the raw material A according to claim 6 or 14, the raw material A comprising a compound of formula I and a compound of formula I';
2) separating the raw material A by column chromatography to give the compound of formula I; and
3) conducting hydroxyl deprotection to the compound of formula I to give purified lanosterol;
wherein the crude product of lanosterol comprises lanosterol and dihydrolanosterol;
the hydroxyl-protecting agent is a silyl ether-based protecting agent.

19. The method according to claim 18, wherein the silyl ether-based protecting agent is selected from trimethylchlorosilane, triethylchlorosilane, tri-tert-butylchlorosilane, *tert-*butyldiphenylchlorosilane, triisopropylchlorosilane, dimethylisopropylchlorosilane, *tert-*butyldimethylchlorosilane, methyldiisopropylchlorosilane, triisopropylchlorosilane, and *tert-*butyldimethylsilyl trifluoromethanesulfonate.

20. The preparation method according to any one of claims 3 to 5, the separation and purification method according to claim 7 or 8, or the method according to claim 18 or 19, wherein the reaction of lanosterol with the hydroxyl-protecting agent is conducted in the presence of an organic solvent;
preferably, the organic solvent is *N*,*N-*dimethylformamide or dichloromethane.

21. The preparation method according to any one of claims 3 to 5, the separation and purification method according to claim 7 or 8, or the method according to claim 18 or 19, wherein the reaction of lanosterol with the hydroxyl-protecting agent is conducted in the presence of a deacid reagent;
preferably, the deacid reagent is an organic base or an inorganic base;
more preferably, the deacid reagent is pyridine, imidazole, diisopropylamine, triethylamine, triethanolamine, potassium carbonate, or sodium carbonate.

22. The preparation method according to any one of claims 3 to 5, the separation and purification method according to claim 7 or 8, or the method according to claim 18 or 19, wherein the reaction of lanosterol with the hydroxyl-protecting agent is conducted at a temperature of 25-120 °C;
preferably, the reaction of lanosterol and the silyl ether-based protecting agent is performed at a temperature of 60-85 °C.

23. The use according to claim 11 or 12 or the method according to claim 18 or 19, wherein the column chromatography is silica gel column chromatography;
preferably, the specification of the silica gel selected for the silica gel column chromatography is 100-200 mesh, 200-300 mesh, or 300-400 mesh;
preferably, the eluent used for the silica gel column chromatography is one or more of petroleum ether, *n*-heptane, *n*-hexane, dichloromethane, and ethyl acetate;
more preferably, aqueous ammonia is added to the eluent used for the silica gel column chromatography.

24. The use according to claim 10 or the method according to claim 18 or 19, wherein the hydroxyl deprotection is conducted in the presence of one or more of acetic acid, tetraalkylammonium fluoride, trifluoroacetic acid, or hydrochloric acid;
preferably, the hydroxyl deprotection is conducted in the presence of tetrabutylammonium fluoride.
